# EUROPEAN PATENT APPLICATION

(11) **EP 1 994 872 A1**
(43) Date of publication of application: **26.11.2008**
(21) Application number: 06798254.6
(22) Date of filing: 22.09.2006
(51) Int. Cl.: A61B 1/00, G02B 23/24

(54) **ENDOSCOPE AND METHOD OF PRODUCING INSERTION SECTION OF ENDOSCOPE**

(30) Priority: 20.02.2006 JP 2006042783
(71) Applicant: Olympus Corporation, Tokyo 151-0072 (JP)
(72) Inventor: KITAGAWA, Hideya, . (JP); NEMOTO, Shigeru, . (JP)
(74) Representative: Winter, Brandl, Fürniss, Hübner Röss, Kaiser, Polte Partnerschaft Patent- und Rechtsanwaltskanzlei
(86) International application number: PCT/JP2006/318866
(87) International publication number: WO 2007/097061

(57) **Abstract**

A bending pipe (52) of a bending portion of an endoscope includes a plurality of node rings (62) each formed into a ring-like shape and arranged in an axial direction of an insertion section in a juxtaposed state, and connection parts (64) for connecting the node rings to each other so as to allow the node rings to be flexibly bent with respect to each other. The node ring includes connection arcuate divisions (72) formed by dividing the node ring into a plurality of pieces, a joint (74a) provided at ends of the connection arcuate divisions and connecting the connection arcuate divisions to each other so as to allow the divisions to be movable with respect to each other in a circumferential direction, and guides (82, 84) provided at ends (74b) of the connection arcuate divisions on the released side, causing these ends to be in contact with each other, and forming the plural connection arcuate divisions into the ring-shaped node ring.

## Description

### Technical Field

The present invention relates to an endoscope used for various objects in, for example, the medical and industrial fields and the like, and a method of manufacturing an insertion section of such an endoscope.

### Background Art

In, for example, Jpn. Pat. Appln. KOKAI Publication No. 10-94514, an insertion section of an endoscope in which a bending pipe of a bending portion is integrally formed of a resin material is disclosed. In the bending pipe, a plurality of node rings are jointed to each other by joints so as to be extended in the axial direction.

In general, in an insertion section of an endoscope, particularly in a bending portion, in order to joint metallic node rings to each other so as to allow them to be turnable in sequence, complication and difficulty in workpiece fabrication and assembly is a problem. In order to solve the problem, there is disclosed a bending pipe in, for example, Jpn. Pat. Appln. KOKAI Publication No. 10-94514, in which many problems in the actual manufacture are included. In manufacturing the bending pipe disclosed in Jpn. Pat. Appln. KOKAI Publication No. 10-94514 by injection molding, the die structure requires, for example, dies of the outer circumference to be divided into a plurality of pieces, and liner dies of the inner circumference which are set inside the dies of the outer circumference. Thus, the die structure becomes complicated. Furthermore, the longer the length of the bending pipe becomes, the more difficult mold release of the liner dies of the inner circumference becomes. Further, a pin-shaped liner die for forming the wire reception part becomes very fine, and it is very difficult to hold the fine die. When the bending pipe is not formed by molding but by removal fabrication, fabrication of the wire reception part becomes very difficult because of the minuteness thereof.

### Disclosure of Invention

The present invention has been contrived to solve the problem described above, and an object thereof is to provide an endoscope in which an insertion section such as a bending portion is easily manufactured, and a method of manufacturing an insertion section of such an endoscope.

In order to solve the problem described above, an endoscope according to the present invention includes a long and thin insertion section, and an operation section provided at a proximal end of the insertion section. Further, the endoscope according to the present invention is characterized in that the insertion section includes a tubular body including a plurality of node rings each formed into a ring-like shape and arranged in an axial direction of the insertion section in a juxtaposed state, and connection parts for connecting the node rings to each other so as to allow the node rings to be flexibly curved with respect to each other, and an outer sheath for covering the tubular body, and the node ring includes connection arcuate divisions formed by dividing the node ring in a circumferential direction into a plurality of pieces, a joint provided between ends of the connection arcuate divisions and connecting the connection arcuate divisions to each other so as to allow the divisions to be openable and closable with respect to each other, and abutting parts each formed at ends of the connection arcuate divisions on the released side, and are brought into contact with each other.

### Brief Description of Drawings

FIG. 1 is a schematic view showing an endoscope according to a first embodiment of the present invention;
FIG. 2 is a schematic front view showing that a bending pipe of a bending portion of an insertion section of the endoscope according to the first embodiment can be disassembled into two connection arcuate divisions;
FIG. 3 is a schematic perspective view showing the bending pipe of the bending portion of the insertion section of the endoscope according to the first embodiment;
FIG. 4 is a schematic perspective view showing the bending pipe of the bending portion of the insertion section of the endoscope according to the first embodiment;
FIG. 5 is a schematic perspective view showing a flexible pipe of a flexible portion of the insertion section of the endoscope according to the first embodiment;
FIG. 6 is a schematic perspective view showing the flexible pipe of the flexible portion of the insertion section of the endoscope according to the first embodiment;
FIG. 7 is a schematic front view showing that a bending pipe of a bending portion of the insertion section of the endoscope according to the first embodiment can be disassembled into three connection arcuate divisions; and
FIG. 8 is a schematic perspective view showing an insertion section tubular body in which a bending portion and flexible portion of an insertion section of an endoscope according to a second embodiment are formed integral with each other.

### Best Mode for Carrying Out the Invention

The best mode for carrying out the present invention will be described below with reference to the accompanying drawings.

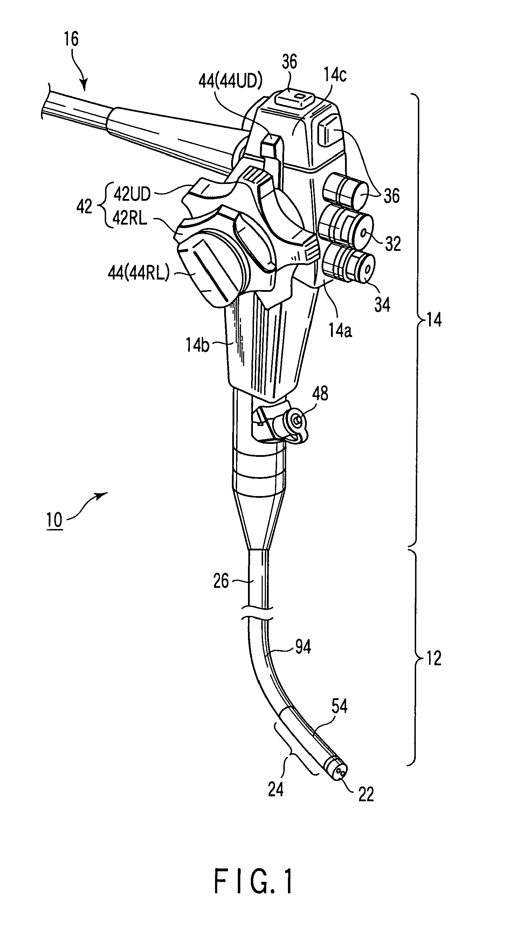

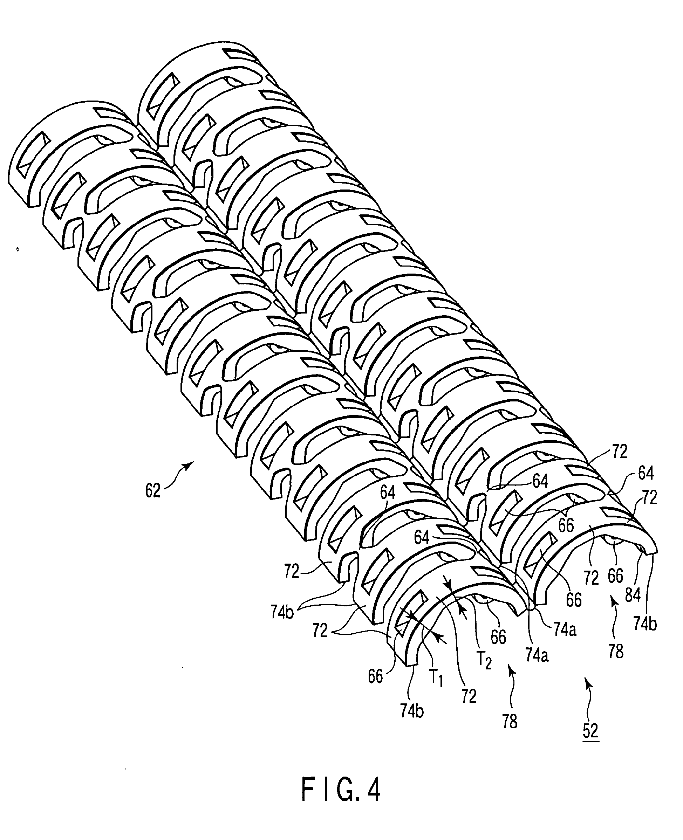

A first embodiment will be described below with reference to FIGS. 1 to 6.

As shown in FIG. 1, an endoscope 10 includes a long and thin insertion section 12, an operation section 14 provided at a proximal end part of the insertion section 12, and a universal cable 16 extended from the operation section 14.

The insertion section 12 includes a distal end hard portion 22, a bending portion 24 provided at a proximal end part of the distal end hard portion 22, and a flexible portion 26 provided at a proximal end part of the bending portion 24. A proximal end part of the flexible portion 26 is connected to the operation section 14. Although not shown, an observation optical system, image pickup device, illumination optical system, air supply/water supply nozzle, forceps exit, and the like are provided in the distal end hard portion 22.

The operation section 14 includes an operation section main body 14a, grip 14b, and switch cover 14c.

In the operation section main body 14a, a suction control valve 32, air supply/water supply valve 34, and remote switches 36 are provided in, for example, a juxtaposed state. A part of the remote switches 36 is provided in the switch cover 14c. A forceps plug 48 is detachably attached to the grip 14b on the insertion section 12 side of the operation section 14.

The suction control valve 32 is used when the suction pipe line (not shown) is changed over. The air supply/water supply valve 34 is used to supply a liquid when an objective lens of the observation optical system is washed or to supply air or water when the used liquid is blown away. The remote switches 36 are used when an image signal picked up by the image pickup device is subjected to desired processing.

Further, functions of, for example, enlarging an image observed by the endoscope 10, and shooting a picture of the image are appropriately assigned to the remote switches 36.

Angle knobs 42 (first and second bending operation knobs 42UD and 42RL) and bending fixing levers 44 (first and second engagement levers 44UD and 44RL) which are formed of hard resin material respectively are attached to the operation section 14.

The first bending operation knob 42UD is operated when the bending portion 24 of the insertion section 12 is bent in the vertical direction. The second bending operation knob 42RL is operated when the bending portion 24 of the insertion section 12 is bent in the lateral direction perpendicular to the vertical direction. The first engagement lever 44UD is operated when the first bending operation knob 42UD is fixed in a desired state. Specifically, the first engagement lever 44UD is used to keep the bending portion 24 in a state where it is bent in the vertical direction. The second engagement lever 44RL is operated when the second bending operation knob 42RL is fixed in a desired state. Specifically, the second engagement lever 44RL is used to keep the bending portion 24 in a state where it is bent in the lateral direction.

Proximal ends of a pair of operation wires (not shown) are connected to each of the first bending operation knob 42UD and second bending operation knob 42RL. Distal ends of these operation wires are fixed to a distal end of the bending pipe 52 of the bending portion 24 to be described later.

The universal cable 16 is covered with a resin material such as polyurethane. A connector (not shown) formed of a hard resin material is attached to an end on the farther side of the operation section 14 with respect to the universal cable 16.

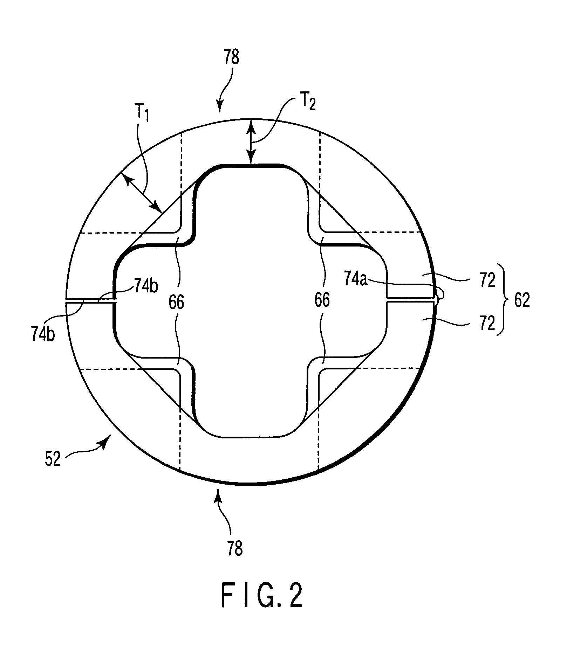

The bending portion 24 includes a bending pipe 52 shown in FIGS. 2 to 4 and a bending pipe outer sheath 54 (see FIG. 1) provided on the outer circumference of the bending pipe 52. The bending pipe outer sheath 54 is formed of, for example, a heat-shrinkable tube that shrinks when heated.

As shown in FIGS. 2 to 4, the bending pipe 52 includes a plurality of node rings (bending pieces) 62 each having a ring-like shape or substantially cylindrical shape and connection parts 64 for jointing these node rings 62 to each other in the axial direction of the bending pipe 52. The node ring 62 has been described as having a ring-like shape or substantially cylindrical shape, but when it is described as simply having a ring-like shape, it is to include various similar shapes such as a substantially cylindrical shape or a cylindrical shape. This also applies to the connection arcuate divisions 72 to be described later.

Each of a pair of first connection parts 64 for connecting a predetermined first node ring 62 and a second node ring 62 adjacent to the first node ring 62 to each other is arranged in a position of about 0^{°} and a position of about 180^{°} with respect to the central axis of the bending pipe 52. In this case, the position of about 0^{°} implies a position corresponding to a formation position of one of four wire reception parts 66 arranged in the circumferential direction with respect to the central axis of the bending pipe 52 to be described later shown in FIG. 2. The position of about 180° is automatically determined every time the position of about 0^{°} is determined.

Further, a pair of second connection parts 64 for connecting the second node ring 62 and a third node ring 62 adjacent to the second node ring 62 to each other are arranged in positions of about 90^{°} and about 270^{°}, respectively, with respect to the central axis of the bending pipe 52. Further, a pair of third connection parts 64 for connecting the third node ring 62 and a fourth node ring 62 adjacent to the third node ring 62 to each other are arranged in positions of about 0^{°} and about 180^{°}, respectively, with respect to the central axis of the bending pipe 52. In this manner, the node rings 62 are connected in sequence by the connection parts 64.

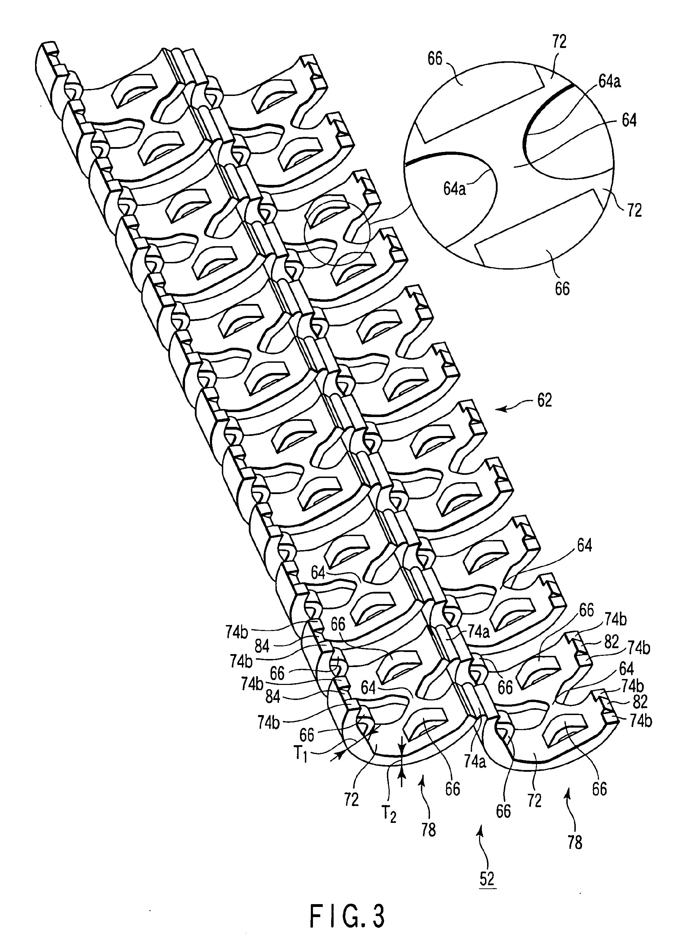

Incidentally, as shown in FIG. 3 in an enlarging manner, in each of a plurality of connection parts 64 between the first node ring 62 and the second node ring 62 (between the connection arcuate divisions 72 adjacent to each other in the axial direction of the bending pipe 52), a pair of circumferential edge parts (edge end surfaces) 64a are formed into curved surfaces in which no flat part is present. More specifically, a pair of edge parts 64a of the connection part 64 are formed into a substantially arched shape. Accordingly, the connection parts 64 smoothly connect the connection arcuate divisions 72 in the axial direction of the bending pipe 52. Each connection part 64 is each bent at the center of each connection part 64 at which the thickness of each connection part 64 is minimized.

Further, in each node ring 62, four wire reception parts 66 are formed in a state where they are cut and bent toward the inside. These wire reception parts 66 are respectively formed in positions of about 0^{°}, about 90^{°}, about 180°, and about 270° with respect to the central axis of the bending pipe 52. Each wire reception part 66 is projected toward the inner peripheral side, made to communicate with adjacent reception parts 66 in the axial direction of the bending pipe 52, and is provided with an opening communicating with the outer circumferential side of the node ring 62.

Each node ring 62 includes connection arcuate divisions 72 which form a ring-shape when a plurality of them are connected to each other, and a joint 74a for connecting the connection arcuate divisions 72 to each other. Further, each node ring 62 includes one end part 74b and the other end part 74b which are formed on the side of the node ring 62 on which the joint 74a for connecting the connection arcuate divisions 72 to each other when a plurality of connection arcuate divisions 72 are connected to each other so as to be formed into a ring-shape is not formed, i.e., on the free side of the connection arcuate divisions 72, as an abutting part, the one end part 74b and the other end part 74b abutting on each other. Each connection arcuate division 72 is formed into a semicircle (semicicumference). That is, in this embodiment, a case where one node ring 62 is constituted of two connection arcuate divisions 72 formed when the node ring 62 is divided into two parts in the circumferential direction will be described.

Each connection arcuate division 72 has an appropriate thickness in the radial direction of the bending pipe 52 and an appropriate thickness in the axial direction thereof. Although the outer circumferential surface of each node ring 62 is formed substantially circular, the inner circumferential surface thereof is not formed circular. Particularly, positions of the node ring 62 in which the connection parts 64 or wire reception parts 66 are arranged are formed thick for reinforcement. The thickness T₁ of the above positions is, for example, 2 mm. A position shifted from the position in which the connection part 64 is arranged by 45^{°} is formed thinner than the position in which the connection part 64 is arranged. The thickness T₂ of this position is, for example, 1 mm. These thicknesses are smoothly changed. Accordingly, buckling resistance of the bending pipe 52 can be improved, and flexibility thereof can be secured.

The joint 74a is provided between one end of the first connection arcuate division 72 which forms one node ring 62 in cooperation with another connection arcuate division to be paired with the first connection arcuate division 72 and the other end of the second connection arcuate division 72. That is, one end of the first connection arcuate division 72 and the other end of the second connection arcuate division 72 are connected to each other by the joint 74a, thereby forming a node ring 62 (see FIG. 2) having a ring-like shape. The joints 74a are formed into hinges aligned in the axial direction of the bending pipe 52. The joint 74a of the first node ring 62 and the joint 74a of the second node ring 62 adjacent to the first node ring 62 are formed on the same axis. Accordingly, the connection arcuate division 72 can be opened or closed in a direction perpendicular to the axial direction of the node ring 62 with the joints 74a being the axis of the opening or closing motion.

According to the flow analysis of the case where polypropylene (PP) which is a polyolefin-based material is used as a polymeric material, each joint 74a may be formed so as to allow it to have a thickness of, for example, about 10% to 25% (for example, 0.25 mm) of the minimum thickness (for example, 1 mm) of the connection arcuate division 72 in the radial direction. The thickness within this range can make the fluidity right and maintain the flexibility of the joint 74a.

Accordingly, in the bending pipe 52, the respective connection arcuate divisions 72 are connected to each other by the joint 74a in the radial direction, and the respective connection arcuate divisions 72 are conncted to each other by the connection parts 64 in the axial direction. Therefore, the bending pipe 52 is constituted of a pair of bending pipe divisions 78 which is formed into a semicylindrical shape in a state where a plurality of connection arcuate divisions 72 are aligned in the axial direction by connection parts 64, and the joints 74a for jointing these bending pipe divisions 78 to each other.

As shown in FIG. 3, a first positioning guide 82 is formed at the other released end (abutting part) 74b of the first connection arcuate division 72. A second positioning guide 84 is formed at the one released end (abutting part) 74b of the second connection arcuate division 72. These first and second positioning guides 82 and 84 prevent the other end 74b of the first connection arcuate division 72 and the one end 74b of the second connection arcuate division 72 from being deviated from each other when these ends 74b are brought into contact with each other.

The first positioning guide 82 is made to protrude from the surface of the other end 74b of the first connection arcuate division 72. The surface of the other end 74b of the first connection arcuate division 72 is made to protrude in the circumferential direction particularly higher on the inner circumferential surface side than on the outer circumferential surface side. Further, the first positioning guide 82 is formed in such a manner that the cross section thereof is substantially triangular.

The second positioning guide 84 is made to be indented from the surface of the one end 74b of the second connection arcuate division 72. The surface of the one end 74b of the second connection arcuate division 72 is made to be indented in the circumferential direction particularly deeper on the inner circumferential surface side than on the outer circumferential surface side. The indented part of the second positioning guide 84 is formed in such a manner that the cross section thereof is substantially triangular. Accordingly, when the surface of the other end 74b of the first connection arcuate division 72 and the surface of the one end 74b of the second connection arcuate division 72 are brought into contact with each other so as to form a ring-like shape, the first positioning guide 82 is engaged with the second positioning guide 84. When the first positioning guide 82 and the second positioning guide 84 are engaged with each other, these positioning guides 82 and 84 prevent the first connection arcuate division 72 and the second connection arcuate division 72 from moving in the radial direction and in the axial direction.

The bending pipe 52 having the structure described above is formed by injection molding using a polypropylene (PP) resin as a material. The bending pipe divisions 78 are integrally formed in the opened state together with the joints 74a. Accordingly, the bending pipe 52 is easily released from the molding die. The wire reception parts 66 are formed by using a die having, for example, a pinch-off structure.

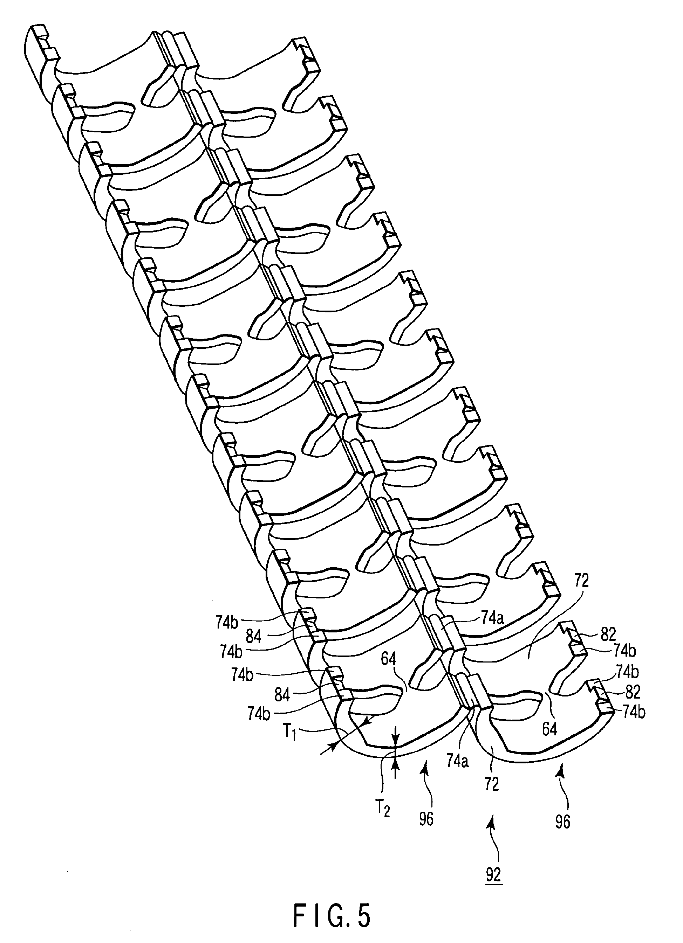

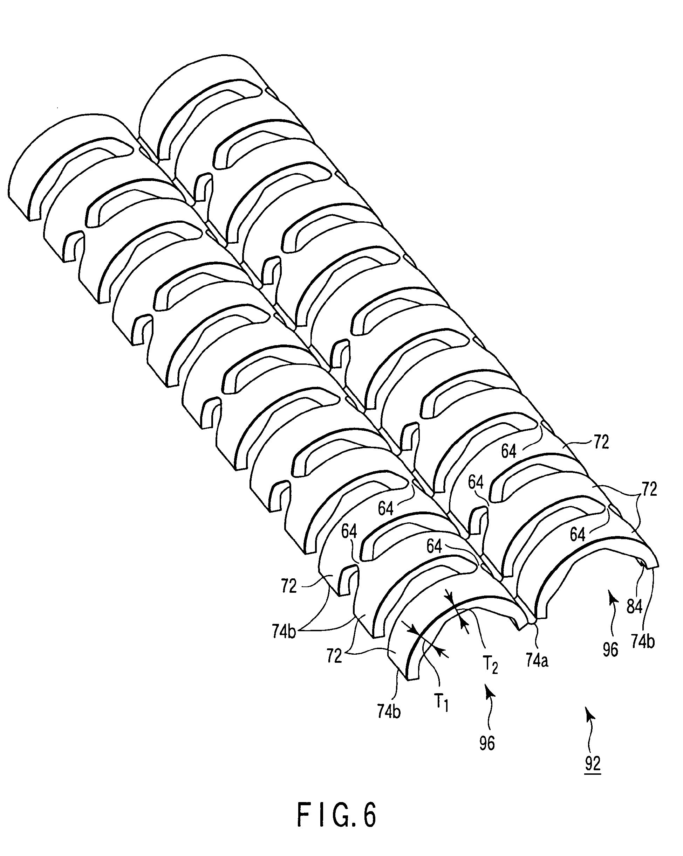

The flexible portion 26 includes a flexible pipe 92 shown in FIGS. 5 and 6, and a flexible pipe outer sheath 94 (see FIG. 1) provided on the outer circumference of the flexible pipe 92. The flexible pipe 92 has the same configuration as the bending pipe 52 except that the wire reception parts 66 of the bending pipe 52 are not formed.

The flexible pipe 92 is formed by injection molding using a polypropylene (PP) resin as is the case with the bending pipe 52. As for manufacture of the flexible pipe 92, like the bending pipe divisions 78 of the bending pipe 52, a pair of flexible pipe divisions 96 are integrally formed in the opened state together with the joints 74a. Accordingly, even the flexible pipe 92 having long members exceeding one meter can be easily released from the molding die.

Next, a method of manufacturing the insertion section 12 of the endoscope 10 according to this embodiment will be described below.

A pair of bending pipe divisions 78 constituting the bending pipe 52 are opened with respect to each other. Wires are passed through the wire reception parts 66 of the connection arcuate divisions of the bending pipe divisions 78. Distal ends of the wires are fixed to the distal ends of the bending pipe divisions 78 (i.e., the bending pipe 52).

Distal ends of the observation optical system, illumination optical system, air supply/water supply tube, and treatment tool passing channel (these are hereinafter referred to as accommodation members) are fixed to the distal end hard portion 22 shown in FIG. 1. Further, the accommodation members are arranged in the bending pipe divisions 78 from the opening of the bending pipe 52 in the state where the bending pipe 52 is opened in the direction perpendicular to the axial direction of the insertion section 12 so as to allow the bending pipe 52 to show a semicylindrical shape, i.e., from the opening formed between the other end and one end of the first and second connection arcuate divisions 72.

The first and second positioning guides 82 and 84 of the first and second connection arcuate divisions 72 are engaged with each other. Then, the other end 74b of the first connection arcuate division 72 and the one end 74b of the second connection arcuate division 72 which are to be brought into contact with each other by the engagement of the guides 82 and 84, are brought into contact with each other, and the abutting parts 74b are connected to each other by jointing them using an adhesive or by welding. That is, each node ring 62 is formed. Thus, the bending pipe 52 is formed. At this time, the accommodation members are already passed through the inside of the bending pipe 52.

Likewise, in the flexible pipe divisions 96, the accommodation members are arranged in the divisions 96 from the opening of the divisions 96. Furthermore, operation wires are also arranged together with the accommodation members. The flexible pipe 92 is formed in the same manner as the node ring 62. At this time, the accommodation members and wires are already passed through the inside of the flexible pipe 92.

Then, the bending pipe 52 and the flexible pipe 92 are positioned in a predetermined state. Thereafter, the bending pipe outer sheath 54 is disposed on the outer circumference of the bending pipe 52, and the flexible pipe outer sheath 94 is disposed on the outer circumference of the flexible pipe 92.

As described above, according to this embodiment, the following can be said.

The bending pipe divisions 78 of the bending pipe 52 or the flexible pipe divisions 96 of the flexible pipe 92 can be integrally formed in a state where the divisions 78 or 96 are opened so as to allow them to show a semicylindrical shape together with the joints 74a by injection molding. Accordingly, release of the molding die can be made easy, and hence even the flexible pipe 92 having a very large length can be easily formed.

In the state where the bending pipe 52 is opened in a semicylindrial form, wires can be passed through the wire reception parts 66 of each node ring 62. Accordingly, wires can be easily passed through the wire reception parts 66. Therefore, the manufacturing work of the bending portion 24 can be performed easily.

After the accommodation members are arranged in the bending pipe 52 and the flexible pipe 92 in the state where the pipes 52 and 92 are opened in a semicylindrical shape, the bending pipe 52 and the flexible pipe 92 can be formed into a pipe-like shape. Accordingly, a work for passing the accommodation members through the bending pipe 52 and the flexible pipe 92 is not required, and the manufacturing work of the bending portion 24 and the flexible portion 26 can be performed easily.

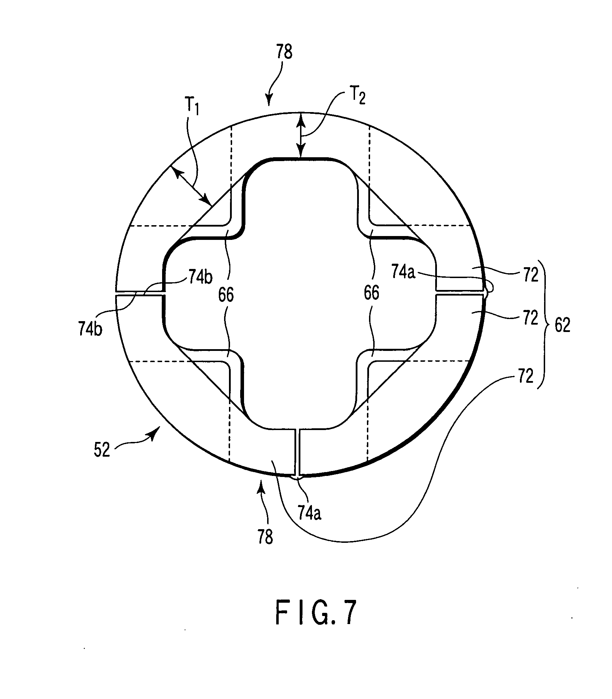

Incidentally, in this embodiment, the node ring 62 in the state where the two connection arcuate divisions 72 are connected to each other by the joint 74a has been described. Further, each node ring 62 may also be formed by connecting three connection arcuate divisions as shown in FIG. 7. In this case, each node ring 62 is formed into a circular shape by a connection arcuate division 72 having a substantially semicircular shape, and a pair of connection arcuate divisions 72 each having a substantially quarter-circular shape. Of these divisions, one of the substantially quarter-circular connection arcuate divisions 72 has joints 74a at both ends thereof. One end of the connection arcuate division 72 which is one of the substantially quarter-circular connection arcuate divisions 72 is connected to an end of the substantially semicircular connection arcuate division 72 by the joint 74a. The other end of the connection arcuate division 72 which is one of the substantially quarter-circular connection arcuate divisions 72 is connected to an end of the other connection arcuate division of the substantially quarter-circular connection arcuate divisions 72 by the joint 74a. That is, each node ring 62 is not limited to a node ring formed by connecting two connection arcuate divisions 72 having the same shape to each other by a joint 74a. Each node ring 62 may also be formed by connecting a plurality of connection arcuate divisions 72 having different shapes by joints 74a.

Further, although not shown, a node ring 62 may also be constituted of four connection arcuate divisions 72. In this case, each node ring 62 is formed into an annular (circular) shape by arranging four substantially quarter-circular connection arcuate divisions 72 in the circumferential direction of a circular shape.

Further, in this embodiment, disposing a bending pipe outer sheath 54 on the circumference of the cuved pipe 52 has been described. However, a reticulate pipe (braid) (not shown) may also be provided between the bending pipe 52 and the bending pipe outer sheath 54. Further, a reticulate pipe (braid) (not shown) may also be provided between the flexible pipe 92 and the flexible pipe outer sheath 94.

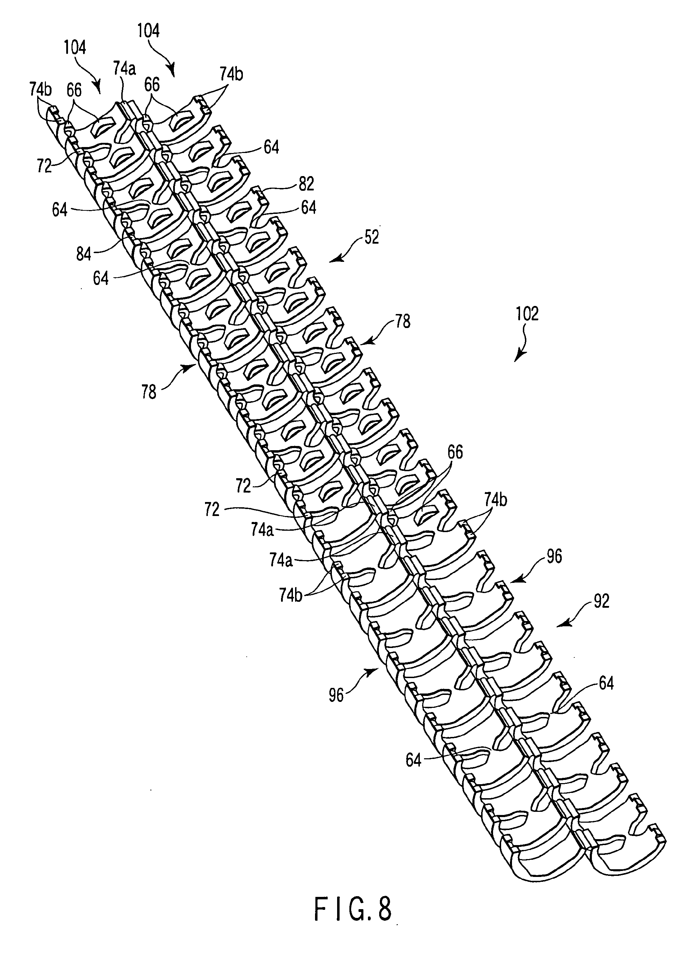

Next, a second embodiment will be described below with reference to FIG. 8. This embodiment is a modified example of the first embodiment, and the same members as those described in the first embodiment are denoted by the same reference symbols and detailed description of them will be omitted.

As shown in FIG. 8, an insertion section 12 includes an intra-insertion section tubular body 102. This tubular body 102 is a member in which the bending pipe 52 and the flexible pipe 92 which are described in the first embodiment are formed integral with each other. The proximal end of the bending pipe 52 and the distal end of the flexible pipe 92 are jointed to each by a connection part 64.

Specifically, the intra-insertion section tubular body 102 includes connection arcuate divisions 72 having wire reception parts 66 in a position corresponding to the bending portion 24, and connection arcuate divisions 72 having no wire reception parts 66 in a position corresponding to the flexible portion 26. In other words, a pair of insertion section divisions 104 in which flexible pipe divisions 96 are connected to each other by the connection parts 64, are connected to the proximal ends of the bending pipe divisions 78 by the joint 74a.

Incidentally, in this embodiment, connection parts 64 are not provided in positions opposed to each other, but are provided between adjacent node rings 62 as shown in FIG. 8. Needless to say, connection parts 64 may also be provided in positions opposed to each other as described in the first embodiment.

A method of manufacturing the insertion section 12 of an endoscope 10 according to this embodiment will be described below.

The insertion section divisions 104 constituting the intra-insertion section tubular body 102 are opened with respect to each other. Wires are passed through the wire reception parts 66 of the connection arcuate divisions 72 of the bending pipe divisions 78. Distal ends of these wires are fixed to the distal ends of the bending pipe divisions 78 (that is, the bending pipe 52).

Distal ends of the observation optical system, illumination optical system, and treatment tool passing channel (these are hereinafter referred to as accommodation members) are fixed to the distal end hard portion 22 shown in FIG. 1. Further, the accommodation members are arranged in the insertion section divisions 104 from the opening of the intra-insertion section tubular body 102 formed in the axial direction of the insertion section 12.

First and second guides 82 and 84 of the connection arcuate divisions 72 are engaged with each other. Then, the other end 74b of the first connection arcuate division 72 and the one end 74b of the second connection arcuate division 72 which are to be brought into contact with each other by the engagement of the guides 82 and 84, are brought into contact with each other, and the abutting parts 74b are connected to each other by jointing them using an adhesive or by welding. That is, each node ring 62 is formed. Thus, the intra-insertion section tubular body 102 is formed. At this time, the accommodation members are already passed through the inside of the intra-insertion section tubular body 102.

Thereafter, a bending pipe outer sheath 54 is disposed on the circumference of a position corresponding to the bending pipe 52, and a flexible pipe outer sheath 94 is disposed on the circumference of a position corresponding to the flexible pipe 92.

As described above, according to this embodiment, the following can be said.

Since the intra-insertion section tubular body 102 is formed by integrally forming the bending pipe 52 of the bending portion 24 and the flexible pipe 92 of the flexible portion 26, the accommodation members can be easily arranged inside the insertion section divisions 104. Accordingly, the bending portion 24 and the flexible portion 26 can be manufactured by one work, and hence the assembling facility of the insertion section 12 of the endoscope 10 can be largely improved.

Incidentally, in the embodiments, description is given on the assumption that the distal ends of the operation wires are fixed to the distal end of the bending pipe 52 of the bending portion 24. However, the present invention is not limited to this, and when the distal ends of the operation wires are fixed to the proximal end side of the distal end hard portion 22, curving operations can be performed in the same manner.

Further, the material of the bending pipe 52 and the flexible pipe 92 is not limited to the polyolefin-based material of the polymeric materials, and elastomeric materials such as a fluoric elastomer, acrylic elastomer, and silicone-based elastomer can also be used.

Some embodiments have been specifically described above with reference the drawings. However, the present invention is not limited to the embodiments described above, and includes all the implementation items performed within a scope not deviating from the gist of the invention.

### Industrial Applicability

According to the present invention, it is possible to provide an endoscope in which an insertion section such as a bending portion is easily manufactured, and a method of manufacturing an insertion section of such an endoscope.

## Claims

1. An endoscope comprising a long and thin insertion section, and an operation section provided at a proximal end of the insertion section, **characterized in that**
the insertion section comprises:
a tubular body including a plurality of node rings each formed into a ring-like shape and arranged in an axial direction of the insertion section in a juxtaposed state, and connection parts for connecting the node rings to each other so as to allow the node rings to be flexibly bent with respect to each other; and
an outer sheath for covering the tubular body; and
the node ring comprises:
connection arcuate divisions formed by dividing the node ring in a circumferential direction into a plurality of pieces;
a joint provided between ends of the connection arcuate divisions and connecting the connection arcuate divisions to each other so as to allow the divisions to be openable and closable with respect to each other; and
abutting parts each formed at ends of the connection arcuate divisions on the released side, and are brought into contact with each other.

2. An endoscope comprising a long and thin insertion section, and an operation section provided at a proximal end of the insertion section, **characterized in that**
the insertion section comprises:
a tubular body including a plurality of node rings each formed into a ring-like shape and arranged in an axial direction of the insertion section in a juxtaposed state, and connection parts for connecting the node rings to each other so as to allow the node rings to be flexibly bent with respect to each other; and
an outer sheath for covering the tubular body; and
the tubular body comprises:
tubular body divisions in which connection arcuate divisions formed by dividing the node ring in a circumferential direction into a plurality of pieces are arranged in an axial direction of the insertion section by the connection parts in a juxtaposed state;
a joint provided between ends of the connection arcuate divisions and connecting the connection arcuate divisions to each other so as to allow the divisions to be openable and closable with respect to each other; and
abutting parts each formed at ends of the connection arcuate divisions on the released side, and are brought into contact with each other.

3. The endoscope according to claim 1 or 2, **characterized in that**
the joint connects the connection arcuate divisions to each other in a hinge-like form.

4. The endoscope according to claim 3, **characterized in that**
the insertion section comprises:
a bending portion to be bending-operated by an operation of the operation section; and
a flexible portion provided at a proximal end of the bending portion; and
the tubular body comprises:
a bending pipe provided in a position corresponding to the bending portion; and
a flexible pipe provided in a position corresponding to the flexible portion.

5. The endoscope according to claim 4, **characterized in that**
the bending pipe integrally includes wire reception parts through which operation wires for bending-operating the bending portion by the operation of the operation section are passed.

6. The endoscope according to claim 5, **characterized in that**
each wire reception part is projected toward a central axis of the insertion section in a state where the wire reception part is cut and bent inwardly from an inner circumferential surface of the node ring.

7. The endoscope according to claim 3, **characterized in that**
the insertion section comprises:
a bending portion to be bending-operated by an operation of the operation section; and
a flexible portion provided at a proximal end of the bending portion; and
the tubular body integrally comprises, in a position corresponding to the bending portion, wire reception parts through which operation wires for bending-operating the bending portion by the operation of the operation section are passed.

8. The endoscope according to claim 7, **characterized in that**
the wire reception part is projected toward a central axis of the insertion section in a state where the wire reception part is cut and bent inwardly from an inner circumferential surface of the node ring.

9. The endoscope according to claim 3, **characterized in that**
the connection parts are each arranged between the node ring and another node ring adjacent to the node ring at positions substantially symmetrical with respect to a central axis of the insertion section in a pair.

10. The endoscope according to claim 9, **characterized in that**
the connection parts are each arranged between another node ring and still another node ring adjacent to another node ring at positions each shifted from the positions arranged between the node ring and another node ring by 90°.

11. The endoscope according to claim 9, **characterized in that**
in each connection part, a pair of edge end surfaces in the circumferential direction are formed into curved surfaces.

12. The endoscope according to claim 11, **characterized in that**
the edge end surfaces of the connection part are each formed into a substantially arched shape.

13. The endoscope according to claim 1 or 2, **characterized in that**
in the connection arcuate divisions formed by dividing the node ring into a plurality of pieces, two of the connection arcuate divisions are connected to each other in the circumferential direction by the joint, and
the abutting parts each provided on the connection arcuate divisions are able to be brought into contact with each other or separated from each other by the joint.

14. The endoscope according to claim 13, **characterized in that**
the abutting parts includes:
an indented part provided on one of the connection arcuate divisions to be brought into contact with each other; and
a protruded part provided on the other of the connection arcuate divisions and being able to be engaged with the one connection arcuate division.

15. The endoscope according to claim 14, **characterized in that**
the indented part includes an indented slope provided on the one connection arcuate division, and
the protruded part includes a protruded slope provided on the other connection arcuate division and is able to be engaged with the indented slope.

16. The endoscope according to claim 14, **characterized in that**
the indented part and the protruded part maintain the engagement with each other by means of the outer sheath.

17. The endoscope according to any one of claims 14 to 16, **characterized in that**
the indented part and the protruded part maintain the engagement with each other by being jointed to each other using an adhesive or by welding.

18. The endoscope according to any one of claims 14 to 16, **characterized in that**
the indented part and the protruded part maintain the engagement with each other by fixing means for fixing the indented part and the protruded part to each other.

19. A method of manufacturing an insertion section of an endoscope in which an insertion section to be inserted into a body cavity includes a bending portion and a flexible portion, and which is used to observe the inside of the body cavity by operating the insertion section so as to bend the bending portion by means of operation wires and by using accommodation members passed through the inside of the insertion section and including observation means for observing the inside of the body cavity, **characterized by** comprising:
a step of arranging the accommodation members, in a state where a plurality of divided connection arcuate divisions constituting a bending pipe included in a bending portion are opened, inside the connection arcuate divisions from a direction intersecting an axial direction of the insertion section;
a step of passing operation wires through wire reception parts provided in the connection arcuate divisions in the axial direction;
the above-mentioned steps being performed in random order, and
a step of thereafter closing the connection arcuate divisions so as to constitute each node ring.

20. A method of manufacturing an insertion section of an endoscope in which an insertion section to be inserted into a body cavity includes a bending portion and a flexible portion, and which is used to observe the inside of the body cavity by operating the insertion section so as to bend the bending portion by means of operation wires and by using accommodation members passed through the inside of the insertion section and including observation means for observing the inside of the body cavity, **characterized by** comprising:
a step of arranging the accommodation members, in a state where a plurality of divided connection arcuate divisions constituting a tubular body included in the insertion section are opened, inside the connection arcuate divisions from a direction intersecting an axial direction of the insertion section;
a step of passing operation wires through wire reception parts provided in the connection arcuate divisions in a position corresponding to the bending portion in the axial direction;
the above-mentioned steps being performed in random order, and
a step of thereafter closing the connection arcuate divisions so as to constitute each node ring.
